# FASCICULE DE BREVET EUROPEEN

(11) **EP 0 612 709 B1**
(45) Date de publication et mention de la délivrance du brevet: **06.12.1995**
(21) Numéro de dépôt: 94400274.0
(22) Date de dépôt: 09.02.1994
(51) Int. Cl.: C07C 17/38, C07C 19/08

(54) **Purification du pentafluoroethane**
Reinigung von Pentafluorethan
Purification of pentafluoroethane

(30) Priorité: 24.02.1993 FR 9302119
(43) Date de publication de la demande: 31.08.1994
(73) Titulaire: ELF ATOCHEM S.A., 92800 Puteaux (FR)
(72) Inventeur: Lacroix, Eric, F-69480 Amberieux d'Azergues (FR); Lantz, André, F-69390 Vernaison (FR); Cheminal, Bernard, F-69510 Soucieu-en-Jarrest (FR)
(74) Mandataire: Leboulenger, Jean

(56) Documents cités:
- EP-A- 0 508 631
- FR-A- 2 381 006
- US-A- 5 087 329

## Description

La présente invention concerne la purification du pentafluoroéthane (ci-après F125) contenant du chloro-pentafluoroéthane (F115) et a plus particulièrement pour objet un procédé de purification consistant à soumettre un mélange F125-F115 à des conditions de fluorations telles que le F115 est transformé en hexafluoroéthane (F116).

Le F125 est un H.F.C. (HydroFluoroCarbone) qui peut être utilisé comme substitut au F115 (un CFC ou ChloroFluoroCarbone) dans le domaine de la réfrigération basse température.

Les procédés connus pour l'obtention du F125 comprennent entre autres la fluoration du perchloréthylène ou de ses dérivés fluorés comme le 1,1-difluoro-1,2,2-trichloroéthane (F122), le 1,1-dichloro-2,2,2-trifluoroéthane (F123) et le 1-chloro-1,2,2,2-tétrafluoroéthane (F124), la fluoration du chlorotrifluoroéthylène (F1113) ou la réduction chimique du F115, notamment l'hydrogénolyse de ce dernier. Dans la majorité des cas, ces voies de synthèse du F125 conduisent soit par formation de sous-produits due aux températures élevées nécessaires à l'obtention de rendements élevés en F125 (cas des réactions de fluoration), soit en raison de conversions non quantitatives du produit de départ (cas de l'hydrogénolyse du F115), à un F125 brut qui est pollué par des quantités non négligeables (plusieurs centaines de ppm à plusieurs dizaines de pour cent) de F115.

Comme indiqué dans le brevet US 5 087 329, la séparation des composés F125 et F115 par distillation est très difficile, voire impossible si l'on souhaite obtenir une teneur très faible en F115.

Or, on s'oriente actuellement vers un F125 commercial de grande pureté dont la teneur en F115 serait de l'ordre d'une centaine de ppm. En raison des difficultés rencontrées pour séparer les composés F125 et F115, l'obtention de teneurs aussi faibles en F115 ne parait pas accessible avec un train de distillation de taille réaliste. On recherche donc actuellement des procédés de purification du F125 permettant d'accéder à ces teneurs très faibles en F115.

Le procédé de séparation du F125 et du F115 décrit dans le brevet US 5 087 329 précité fait appel à la distillation extractive consistant, avant distillation, à ajouter au mélange F125-F115 un agent d'extraction constitué par un hydrocarbure fluoré en C₁-C₄ éventuellement hydrogéné et'ou chloré ayant un point d'ébullition compris entre -39°C et +50°C. Comme exemples de tels agents d'extraction, le brevet mentionne le 1,2-dichlorotétrafluoroéthane (F114), le 1,1-dichlorotétrafluoroéthane (F114a), le 1,1,2-trichlorotrifluoroéthane (F113), le 1,1,1-trichlorotrifluoroéthane (F113a), le 2-chloro-1,1,1,2-tétrafluoroéthane (F124), le 2,2-dichloro-1,1,1-trifluoroéthane (F123), le trichlorofluorométhane (F11) et l'octafluorocyclobutane (F-C318).

Dans la demande de brevet EP 508 631 qui décrit la production de composés H.F.C. par réduction chimique en phase liquide de composés chlorés, bromés ou iodés avec un hydrure métallique ou un complexe d'un tel hydrure, il est indiqué que ce procédé peut être intéressant pour purifier certains H.F.C. comme le F125. La purification du F125 n'est pas exemplifiée. Par contre, l'exemple 6 décrit la réduction du F115 par LiAlH₄ dans du diglyme ; le meilleur résultat indique une conversion du F115 en F125 de 60 %.

Il a maintenant été trouvé qu'il est possible de fluorer un mélange F125-F115 pour transformer le F115 en F116 avec une bonne conversion, sans pratiquement altérer le F125. Dès lors, une simple distillation du F116 formé permet d'accéder à un F125 contenant seulement quelques centaines de ppm de F115.

La présente invention a donc pour objet un procédé de purification du F125, caractérisé en ce qu'il consiste à soumettre à une fluoration le mélange F125-F115 à purifier, puis à séparer le F116 formé.

Toute méthode de fluoration connue permettant la conversion du F115 en F116 sans dégrader des quantités trop importantes de F125 peut être mise en oeuvre pour réaliser la première étape du procédé de purification selon l'invention.

La fluoration du F115 en F116 étant relativement difficile, elle nécessite des moyens de fluoration sévères. Bien qu'on puisse opérer en phase liquide avec des agents de fluoration puissants tels que SbF₅, on préfère mettre en oeuvre une fluoration catalytique en phase gazeuse avec HF qui présente l'avantage d'être facilement industrialisable et d'être applicable à un procédé continu, sans nécessiter des régénérations ou des changements de charges catalytiques trop fréquents.

Le catalyseur utilisé pour la fluoration en phase gazeuse peut être choisi parmi tous les catalyseurs massiques ou supportés, connus de l'homme de l'art pour ce type de fluoration. A titre non limitatif, on peut mentionner plus particulièrement :
- les catalyseurs à base de chrome, de nickel et/ou de magnésium supportés sur un matériau compatible avec l'acide fluorhydrique tel que, par exemple, le charbon, l'alumine et le trifluorure d'aluminium,
- des catalyseurs massiques tels que l'alumine, les dérivés du chrome, en particulier un oxyde de chrome III, ou des mixtes à base de chrome et d'un autre constituant comme, par exemple, le nickel, le magnésium ou le zinc.

Sont plus particulièrement préférés pour la mise en oeuvre du procédé selon l'invention les catalyseurs Cr₂O₃ massiques, les catalyseurs Ni-Cr massiques tels que décrits dans la demande de brevet EP 546883 dont le contenu est incorporé ici par référence, et les catalyseurs mixtes à base d'oxydes, halogénures et/ou oxyhalogénures de nickel et de chrome déposés sur un support constitué de fluorure d'aluminium ou d'un mélange de fluorures d'aluminium et d'alumine. Ce dernier type de catalyseur est décrit dans la demande de brevet FR 2 669 022 dont le contenu est incorporé ici par référence.

Pour un catalyseur donné, les conditions opératoires de la fluoration en phase gazeuse du mélange F125-F115 à purifier dépendent de l'objectif souhaité en F115 résiduel et sont généralement les suivantes :
**a)** La température de la réaction est généralement comprise entre 300 et 600°C ; plus elle est élevée, plus grande est la conversion du F115. Cependant, puisque des températures trop élevées peuvent être à l'origine d'une décomposition importante du F125 et peuvent être également préjudiciables à la durée de vie du catalyseur, la gamme des températures préférées est comprise entre 350 et 550°C, et plus particulièrement entre 380 et 480°C.
**b)** Le temps de contact, calculé comme étant le temps de passage des gaz (dans les conditions réactionnelles) à travers le volume de catalyseur en vrac, est compris entre 3 et 300 secondes, et plus particulièrement, entre 15 et 200 secondes. Là aussi, il doit être ajusté en fonction de l'objectif en F115 résiduel recherché. Une augmentation de ce temps de contact abaisse les concentrations en F115 résiduel, mais en contrepartie les volumes de F125 traités sont plus faibles et généralement les réactions parasites de dégradation du F125 sont légèrement augmentées. A la différence de la température, ce paramètre influe peu sur la désactivation du catalyseur ; par conséquent, la marge de manoeuvre sur ce paramètre est plus importante et une optimisation de ce temps de contact permet d'aboutir au meilleur compromis entre teneur en F115 résiduel et productivité de l'installation.
**c)** Le débit d'HF -exprimé pour des raisons pratiques par le rapport molaire HF/organiques (F125 + F115)- dépend de la proportion de F115 dans le brut à traiter. Ce rapport molaire influe sur le produit final purifié (teneur en F115), mais également sur la stabilité du F125 et indirectement sur la durée de vie du catalyseur. Il peut être compris entre 0,05 et 20. Un bon compromis entre une conversion du F115 élevée et des quantités raisonnables d'HF n'ayant pas réagi, réduit cette fourchette aux valeurs 0,5 à 10. Préférentiellement on utilisera un rapport molaire compris entre 0,6 et 5.
**d)** Dans des conditions opératoires susceptibles d'encrasser le catalyseur [faible rapport molaire, températures élevées (>500°C)], il peut être judicieux d'introduire avec les réactifs, de l'oxygène à faible teneur. Cette teneur peut varier selon les conditions opératoires entre 0,02 et 5 % par rapport aux réactifs organiques (pourcentage molaire). Cet oxydant a pour but de réagir avec les "lourds" qui sont à l'origine de l'encrassement du catalyseur.
**e)** La réaction de fluoration selon l'invention peut être menée à la pression atmosphérique ou à une pression supérieure à cette dernière. Pour des raisons pratiques, on opérera généralement dans une zone allant de 0 à 25 bars relatifs.
**f)** Les matériaux utilisés pour la construction de l'installation doivent être compatibles avec la présence d'hydracides tels que HCl et HF ; ils peuvent être choisis en "Hastelloy" ou en "Inconel" qui sont résistants aux milieux corrosifs contenant ces hydracides.
**g)** Selon l'objectif recherché, notamment pour atteindre des teneurs en F115 de l'ordre de la centaine de ppm, il peut s'avérer utile d'utiliser plusieurs réacteurs en série.

Après l'étape de fluoration, le F116 formé et les éventuels sous-produits (trifluorométhane, CO, CO₂) peuvent être facilement séparés du F125, par exemple par simple distillation puisque leurs points d'ébullition sont très éloignés de celui du F125.

Bien que le procédé selon l'invention puisse être appliqué à des mélanges F125-F115 contenant des teneurs élevées en F115 (par exemple jusqu'à 30 %), il est plus particulièrement destiné à la purification d'un F125 brut dont la teneur molaire en F115 n'excède pas 10 %. Par ailleurs, ce F125 brut à purifier peut également contenir, en faibles proportions, des sous-produits résultant de sa synthèse (F123, F124, F143a, F134a, HCl, ...).

La mise en oeuvre du procédé selon l'invention permet d'obtenir du F125 commercial de très grande pureté avec une teneur résiduelle en F115 abaissée, dans certains cas, jusqu'à la centaine de ppm. La fluoration en phase gazeuse permet d'obtenir ce degré de pureté en utilisant des installations industrielles fonctionnant en continu ; cependant, dans certains cas, des variantes telles que des fluorations en phase liquide peuvent s'avérer mieux adaptées à ce problème de purification du F125.

Les exemples suivants illustrent l'invention sans la limiter.

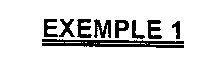

### 1A - PREPARATION ET ACTIVATION DU CATALYSEUR

Dans un évaporateur rotatif, on place 250 ml d'un support contenant en poids 73 % de fluorure d'aluminium et 27 % d'alumine, obtenu dans une étape précédente par fluoration d'alumine GRACE HSA en lit fluidisé vers 300°C à l'aide d'air et d'acide fluorhydrique (concentration volumique de 5 à 10 % de cet acide dans l'air). L'alumine GRACE HSA de départ présente les caractéristiques physico-chimiques suivantes :
- forme : billes de 1-2 mm de diamètre
- surface BET : 220m²/g
- volume poreux : 1,2 cm³/g (pour les rayons de pores compris entre 4 nm et 63 »m)
- teneur en sodium : 600 ppm
On prépare par ailleurs deux solutions aqueuses séparées :
**(a)** solution chromique contenant :

| | |
|---|---|
| - anhydride chromique | 57 g |
| - eau | 41 g |

**(b)** Solution méthanolique contenant :

| | |
|---|---|
| - méthanol | 81 g |
| - eau | 18 g |

Le mélange de ces deux solutions est ensuite introduit, à température ambiante sous pression atmosphérique et en 45 minutes environ, sur le support en agitation. Le catalyseur est alors séché sous courant d'azote, en lit fluidisé, vers 110°C pendant 4 heures.

On charge 100 ml (95 g) de catalyseur sec dans un réacteur tubulaire en INCONEL de diamètre intérieur 27 mm et l'on monte la température à 120°C sous courant d'azote, à pression atmosphérique. On maintient ce traitement pendant une dizaine d'heures puis on remplace progressivement l'azote par de l'acide fluorhydrique en veillant à ce que l'augmentation de température n'excède pas 95°C et, lorsque l'on atteint un ratio molaire HF/N₂ de 50/50, on élève la température à 300°C.

Après disparition des pics d'exothermie, on monte la température à 350°C sous courant d'acide fluorhydrique pur (1 mole/heure) pendant 6 heures.

Le catalyseur est finalement balayé sous courant d'azote avant de démarrer le test catalytique. Les caractéristiques du catalyseur A ainsi séché et activé sont les suivantes :

| - composition chimique (pondérale) | |
|---|---|
| . fluor | 52,0 % |
| . aluminium | 22,0 % |
| . chrome | 14,9 % |
| . oxygène | 11,1 % (complément à 100) |

| - propriétés physiques : | |
|---|---|
| . surface BET | 22,7 m²/g |
| . volume des pores d'un rayon compris entre 4 nm et 63 »m | 0,38 cm³/g |
| . surface des pores de rayon supérieur à 4 nm | 21,9 m²/g |

### 1B - FLUORATION DU F115 DANS UN MELANGE F125-F115

Les performances du catalyseur A dans la fluoration du F115 présent dans un F125 brut ont été testées à pression atmosphérique, sans addition d'oxygène, dans les conditions opératoires et avec les résultats rassemblés dans le tableau 1 suivant.

Le F125 brut de départ est pur à 98,35 % (% molaire), les impuretés majoritaires étant le F115 (1,54 %) et le F124 (550 ppm).

**TABLEAU 1**

| **TEST N°** | **11** | **12** | **13** |
|---|---|---|---|
| ***Conditions opératoires*** : | | | |
| - Température (°C) | 450 | 450 | 500 |
| - Rapport molaire HF/F115+F125 | 1 | 1,1 | 0,9 |
| - Temps de contact (s) | 18,3 | 33,4 | 16,9 |
| - Age du catalyseur (h) | 23 | 47 | 67 |

| ***Résultats*** : | | | |
|---|---|---|---|
| - Taux de transformation global du F115 | 56,5 | 72,7 | 59,1 |
| - Sélectivité en F116 (%) | ∼ 100 | ∼ 100 | ∼ 100 |
| - Taux de transformation global du F125 (%) | 0,14 | 0,16 | 0,3 |
| - Teneur en F115 dans le F125 purifié (% molaire) | 0,67 | 0,42 | 0,63 |

Les sous-produits formés et identifiés, après neutralisation des acides (HF et HCl) par lavage, sont le F23 (CF₃H) et du CO/CO₂.

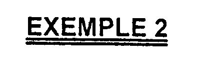

On prépare un catalyseur B à base de nickel et de chrome déposés sur AlF₃. On opère comme à l'exemple 1A, mais en utilisant les deux solutions aqueuses suivantes :
**(a)** solution chromique additionnée de chlorure de nickel contenant :

| | |
|---|---|
| - anhydride chromique | 12,5 g |
| - chlorure de nickel hexahydraté | 29 g |
| - eau | 42 g |

**(b)** Solution méthanolique contenant :

| | |
|---|---|
| - méthanol | 18 g |
| - eau | 33 g |

Après séchage et activation, les caractéristiques du catalyseur B sont les suivantes :

| - composition chimique (pondérale) | |
|---|---|
| . fluor | 64,4 % |
| . aluminium | 27,2 % |
| . nickel | 3,8 % |
| . chrome | 3,3 % |
| . oxygène | 1,3 % (complément à 100) |

| - propriétés physiques : | |
|---|---|
| . surface BET | 35,4 m²/g |
| . volume des pores d'un rayon compris entre 4 nm et 63 »m | 0,47 cm³/g |
| . surface des pores de rayon supérieur à 4 nm | 32,8 m²/g |

Le tableau 2 suivant rassemble les conditions opératoires et les résultats obtenus avec ce catalyseur B dans la fluoration, à pression atmosphérique, du F115 contenu dans le même F125 brut qu'à l'exemple 1B.

**TABLEAU 2**

| **TEST N°** | **21** | **22** | **23** |
|---|---|---|---|
| ***Conditions opératoires*** : | | | |
| - Température (°C) | 450 | 450 | 500 |
| - Rapport molaire HF/F115+F125 | 0,9 | 1 | 1 |
| - Temps de contact (s) | 19,9 | 35,5 | 18,1 |
| - Age du catalyseur (h) | 24 | 48 | 72 |

| ***Résultats*** : | | | |
|---|---|---|---|
| - Taux de transformation global du F115 (%) | 64,3 | 80,5 | 39,6 |
| - Sélectivité en F116 (%) | 95 | 94 | 98 |
| - Taux de transformation global du F125 (%) | < 0,1 | < 0,1 | 0,2 |
| - Teneur en F115 dans le F125 purifié (% molaire) | 0,55 | 0,3 | 0,93 |

Comme à l'exemple 1A, les sous-produits formés et identifiés après lavage, sont le F23 (CF₃H) et du CO/CO₂.

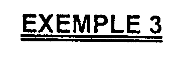

On prépare un catalyseur C selon l'invention dont les teneurs en chrome et en nickel sont sensiblement le double de celles du catalyseur B. On opère comme à l'exemple 1A, mais en utilisant les deux solutions aqueuses suivantes :
**(a)** solution chromique additionnée de chlorure de nickel contenant :

| | |
|---|---|
| - anhydride chromique | 25 g |
| - chlorure de nickel hexahydraté | 58 g |
| - eau | 40 g |

**(b)** Solution méthanolique contenant :

| | |
|---|---|
| - méthanol | 35 g |
| - eau | 30 g |

Après séchage et activation, les caractéristiques du catalyseur C sont les suivantes :

| - composition chimique (pondérale) | |
|---|---|
| . fluor | 58,5 % |
| . aluminium | 25,1 % |
| . nickel | 6,8 % |
| . chrome | 5,6 % |

| - propriétés physiques : | |
|---|---|
| . surface BET | 15,1 m²/g |
| . volume des pores d'un rayon compris entre 4 nm et 63 »m | 0,382 cm³/g |
| . surface des pores de rayon supérieur à 4 nm | 18 m²/g |

Le tableau 3 suivant rassemble les conditions opératoires et les résultats obtenus avec ce catalyseur C dans la fluoration, à pression atmosphérique, du même F125 brut qu'à l'exemple 1B.

**TABLEAU 3**

| **TEST N°** | **31** | **32** | **33** | **34** | **35** |
|---|---|---|---|---|---|
| ***Conditions opératoires*** : | | | | | |
| - Température (°C) | 450 | 450 | 450 | 450 | 450 |
| - Rapport molaire HF/F115+F125 | 1 | 1,1 | 1,2 | 0,8 | 0,6 |
| - Temps de contact (s) | 25 | 101 | 103 | 100 | 107 |
| - Age du catalyseur (h) | 24 | 48 | 94 | 118 | 190 |

| ***Résultats*** : | | | | | |
|---|---|---|---|---|---|
| - Taux de transformation global du F115 (%) | 87 | 98,2 | 99,4 | 97,7 | 96,8 |
| - Sélectivité en F116 (%) | ∼ 100 | ∼ 100 | ∼ 100 | ∼ 100 | ∼ 100 |
| - Taux de transformation global du F125 (%) | < 0,1 | 0,2 | 0,2 | 0,25 | 0,4 |
| - Teneur molaire en F115 dans le F125 purifié | 0,2 % | 270 ppm | 100 ppm | 350 ppm | 600 ppm |

Les sous-produits formés et identifiés après lavage sont les mêmes qu'aux exemples 1 et 2.

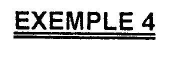

On utilise un catalyseur massique à base d'oxyde de chrome activé de la façon suivante :
On charge 100 ml (134 g) d'oxyde de chrome III dans un réacteur tubulaire en INCONEL de diamètre intérieur 27 mm et l'on monte la température à 120°C sous courant d'azote, à pression atmosphérique. On maintient ce traitement pendant une dizaine d'heures puis on remplace progressivement l'azote par de l'acide fluorhydrique en veillant à ce que l'augmentation de température n'excède pas 95°C et, lorsque l'on atteint un ratio molaire HF/N₂ de 50/50, on élève la température à 300°C.

Après disparition des pics d'exothermie, on monte la température à 350°C sous courant d'acide fluorhydrique pur (1 mole/heure) pendant 15 heures.

Le catalyseur est finalement balayé sous courant d'azote avant de démarrer le test catalytique. Les caractéristiques du catalyseur D ainsi séché et activé sont les suivantes :

| - composition chimique (pondérale) | |
|---|---|
| . fluor | 26,9% |
| . chrome | 53,3 % |
| . oxygène | 19,8 % (complément à 100) |

| - propriétés physiques : | |
|---|---|
| . surface BET | 101 m²/g |
| . volume des pores d'un rayon compris entre 4 nm et 63 »m | 0,13 cm³/g |
| . surface des pores de rayon supérieur à 4 nm | 35,9 m²/g |

Le tableau 4 suivant rassemble les conditions opératoires et les résultats obtenus avec ce catalyseur D dans la fluoration, à pression atmosphérique, du F115 contenu dans le même F125 brut qu'à l'exemple 1B.

**TABLEAU 4**

| **TEST N°** | **41** | **42** | **43** |
|---|---|---|---|
| ***Conditions opératoires*** : | | | |
| - Température (°C) | 450 | 450 | 400 |
| - Rapport molaire HF/F115+F125 | 1 | 1 | 1 |
| - Temps de contact (s) | 24 | 100 | 95 |
| - Age du catalyseur (h) | 19 | 41 | 108 |

| ***Résultats*** : | | | |
|---|---|---|---|
| - Taux de transformation global du F115 (%) | 90,3 | 100 | 91,6 |
| - Sélectivité en F116 (%) | ∼ 100 | ∼ 100 | ∼ 100 |
| - Taux de transformation global du F125 (%) | 1,3 | 2,5 | 1 |
| - Teneur en F115 dans le F125 purifié (% molaire) | 0,15 | traces | 0,13 |

## Revendications

1. Procédé de purification d'un pentafluoroéthane brut contenant du chloropentafluoroéthane, caractérisé en ce qu'il consiste à soumettre le pentafluoroéthane brut à une fluoration catalytique pour transformer le chloropentafluoroéthane en hexafluoroéthane, puis à séparer l'hexafluoroéthane formé.

2. Procédé selon la revendication 1, dans lequel l'hexafluoroéthane formé est séparé par distillation.

3. Procédé selon la revendication 1 ou 2, dans lequel la fluoration est effectuée en phase gazeuse au moyen d'acide fluorhydrique.

4. Procédé selon la revendication 3, dans lequel la fluoration est réalisée à une température comprise entre 300 et 600°C, de préférence entre 350 et 550°C, et plus particulièrement entre 380 et 480°C.

5. Procédé selon la revendication 3 ou 4, dans lequel le temps de contact est compris entre 3 et 300 secondes, de préférence entre 15 et 200 secondes.

6. Procédé selon l'une des revendications 3 à 5, dans lequel le rapport molaire HF/pentafluoroéthane + chloropentafluoroéthane est compris entre 0,05 et 20, de préférence entre 0,5 et 10, et plus particulièrement entre 0,6 et 5.

7. Procédé selon l'une des revendications 3 à 6, dans lequel la fluoration est effectuée à une pression allant de 0 à 25 bars relatifs, de préférence à la pression atmosphérique.

8. Procédé selon l'une des revendications 3 à 7, dans lequel la fluoration est effectuée en présence d'oxygène.

9. Procédé selon l'une des revendications 3 à 8, dans lequel on utilise un catalyseur mixte à base d'oxydes, halogénures et/ou oxyhalogénures de nickel et de chrome déposés sur un support constitué de fluorure d'aluminium ou d'un mélange de fluorure d'aluminium et d'alumine.

10. Procédé selon l'une des revendications 3 à 8, dans lequel on utilise un catalyseur massique Cr₂O₃ ou Ni-Cr.

## Claims

1. Process for the purification of a crude pentafluoroethane containing chloropentafluoroethane, characterized in that it consists in subjecting the crude pentafluoroethane to a catalytic fluorination to convert chloropentafluoroethane to hexafluoroethane, and then in isolating the hexafluoroethane formed.

2. Process according to Claim 1, in which the hexafluoroethane formed is isolated by distillation.

3. Process according to Claim 1 or 2, in which the fluorination is performed in the gas phase by means of hydrofluoric acid.

4. Process according to Claim 3, in which the fluorination is carried out at a temperature of between 300 and 600°C, preferably between 350 and 550°C, and more particularly between 380 and 480°C.

5. Process according to Claim 3 or 4, in which the contact time is between 3 and 300 seconds, preferably between 15 and 200 seconds.

6. Process according to one of Claims 3 to 5, in which the molar ratio HF/pentafluoroethane + chloropentafluoroethane is between 0.05 and 20, preferably between 0.5 and 10, and more particularly between 0.6 and 5.

7. Process according to one of Claims 3 to 6, in which the fluorination is performed at a pressure ranging from 0 to 25 bars gauge, preferably at atmospheric pressure.

8. Process according to one of Claims 3 to 7, in which the fluorination is performed in the presence of oxygen.

9. Process according to one of Claims 3 to 8, in which a mixed catalyst is employed which is based on nickel and chromium oxides, halides and/or oxyhalides which are deposited on a support consisting of aluminium fluoride or of a mixture of aluminium fluoride and alumina.

10. Process according to one of Claims 3 to 8, in which a bulk Cr₂O₃ or Ni-Cr catalyst is employed.

## Patentansprüche

1. Verfahren zur Reinigung von technischem Pentafluorethan, das Chlorpentafluorethan enthält, dadurch gekennzeichnet, daß man das technische Pentafluorethan einer katalytischen Fluorierung unterwirft, um das Chlorpentafluorethan in Hexafluorethan zu überführen, und dann das gebildete Hexafluorethan abtrennt.

2. Verfahren nach Anspruch 1, in dem das gebildete Hexafluorethan mittels Destillation abgetrennt wird.

3. Verfahren nach Anspruch 1 oder 2, in dem die Fluorierung in der Gasphase mittels Fluorwasserstoff durchgeführt wird.

4. Verfahren nach Anspruch 3, in dem die Fluorierung bei einer Temperatur zwischen 300 und 600 °C, vorzugsweise zwischen 350 und 550 °C, insbesondere zwischen 380 und 480 °C, durchgeführt wird.

5. Verfahren nach Anspruch 3 oder 4, in dem die Kontaktzeit 3 bis 300 Sekunden, vorzugsweise 15 bis 200 Sekunden, beträgt.

6. Verfahren nach einem der Ansprüche 3 bis 5, in dem das Molverhältnis HF/Pentafluorethan + Chlorpentafluorethan zwischen 0,05 und 20, vorzugsweise zwischen 0,5 und 10, insbesondere zwischen 0,6 und 5, beträgt.

7. Verfahren nach einem der Ansprüche 3 bis 6, in dem die Fluorierung bei einem Druck von 0 bis 25 bar, insbesondere bei Atmosphärendruck, durchgeführt wird.

8. Verfahren nach einem der Ansprüche 3 bis 7, in dem die Fluorierung in Anwesenheit von Sauerstoff durchgeführt wird.

9. Verfahren nach einem der Ansprüche 3 bis 8, in dem man einen Mischkatalysator auf Basis von Nickel- und Chromoxiden, -halogeniden und/oder -oxyhalogeniden verwendet, die auf einem Trägermaterial aufgebracht sind, das aus Aluminiumfluorid oder einer Aluminiumfluorid/Aluminiumoxid-Mischung besteht.

10. Verfahren nach einem der Ansprüche 3 bis 8, in dem Cr₂O₃ oder Ni-Cr als Katalysator in Masse verwendet.
